# EUROPEAN PATENT APPLICATION

(11) **EP 4 544 988 A1**
(43) Date of publication of application: **30.04.2025**
(21) Application number: 23206130.9
(22) Date of filing: 26.10.2023
(51) Int. Cl.: A61B 5/00, A61B 5/026, G02B 27/48, G06T 7/00

(54) **METHOD, COMPUTER PROGRAM PRODUCT, AND DEVICE FOR ANALYZING A SPECKLE PATTERN FROM BIOLOGICAL TISSUE**

(71) Applicant: Stichting IMEC Nederland, 5656 AE Eindhoven (NL); Imec VZW, 3001 Leuven (BE); Katholieke Universiteit Leuven KU Leuven Research & Development, 3000 Leuven (BE)
(72) Inventor: LORATO, Ilde Rosa, 5653 JG Eindhoven (NL); HERRANZ OLAZABAL, Jorge, 5595 HB Leende (NL)
(74) Representative: AWA Sweden AB

(57) **Abstract**

According to an aspect of the present inventive concept there is provided a method for analyzing a speckle pattern from biological tissue, the method comprising:
receiving a time sequence of images of the speckle pattern, wherein each image of the time sequence of images comprises a plurality of elements; and
for each respective image of the time sequence of images:
applying a digital spatial filter procedure to the image for enhancing areas in the image corresponding to speckles of the speckle pattern, thereby generating a filtered image wherein a morphology of speckles of the speckle pattern is enhanced by compensation for variations in light intensity in the image; and
analyzing the filtered image to determine a speckle pattern value representing an amount of distinguishable speckles of the speckle pattern in at least one region of the filtered image.

## Description

### Technical field

The present disclosure relates to optical monitoring of biological tissue, and more specifically to a method, a computer program product, and a device for analyzing a speckle pattern from biological tissue.

### Background

Speckles are interference patterns generated when a coherent light source, such as a laser, is used to illuminate a surface. Although often regarded as noise in imaging applications, speckles can also provide useful information. When the illuminated surface is static the speckle pattern will be static and form bright and dark dots. However, if the surface presents changes in time, such as movements or scattering changes, the speckle pattern will be perturbated.

In medical applications, Laser Speckle Imaging (LSI) may be used to exploit the blurriness of speckles to extract information from biological tissue, such as tissue perfusion. Images of the speckle pattern may be acquired by an imaging detector, after which the speckles are typically processed to estimate the speckle contrast, which is a measure of the blurriness present in the images. Further, by analyzing the speckle patterns in separate regions of the image, maps of speckle contrast containing information of perfusion may be produced. Another approach used in medical applications is Speckle Plethysmography (SPG). SPG allows for analysis of spatiotemporal changes of the speckle pattern. A time series similar to Photo Plethysmography (PPG) can be obtained, by analyzing how the speckle contrast in the images changes in time. The method used to extract SPG from videos is the same used for LSI, i.e. the speckle contrast.

However, there is a need in the art for further improvements related to optical monitoring of biological tissue and thus to analysis of speckle patterns therefrom.

### Summary

An objective of the present disclosure is to mitigate, alleviate or eliminate one or more deficiencies in the art and disadvantages singly or in any combination. These and other objectives are at least partly met by the invention as defined in the independent claims. Preferred embodiments are set out in the dependent claims.

According to a first aspect there is provided a method for analyzing a speckle pattern from biological tissue, the method comprising:
receiving a time sequence of images of the speckle pattern, wherein each image of the time sequence of images comprises a plurality of elements; and
for each respective image of the time sequence of images:
   applying a digital spatial filter procedure to the image for enhancing areas in the image corresponding to speckles of the speckle pattern, thereby generating a filtered image wherein a morphology of speckles of the speckle pattern is enhanced by compensation for variations in light intensity in the image; and
   analyzing the filtered image to determine a speckle pattern value representing an amount of distinguishable speckles of the speckle pattern in at least one region of the filtered image.

By the term "speckles" is here meant an interference pattern formed when coherent light illuminates a surface. On a static surface, the speckle pattern will be static and form bright and dark dots due to constructive and destructive interference of light. Speckle patterns may be formed when illuminating light from a light source is reflected by the surface, which is referred to as reflection mode. Further, speckle patterns may be formed when illuminating light from the light source is transmitted through the surface, which is referred to as transmission mode. In the present context, light illuminating a surface of the biological tissue may exit at an opposite side of the biological tissue, where a speckle pattern may be formed.

Living biological tissue presents movements of the tissue, caused by for example blood flow through the tissue and contraction and dilatation of blood vessels in the tissue. When living biological tissue is illuminated by coherent light, the speckle pattern is perturbed by this movement, such that the speckle pattern is blurred. The contrast of the speckles is reduced, which may result in fewer distinguishable speckles of the speckle pattern.

It serves to mention that the term plethysmography refers to measurements of changes in volume. By way of example, in conventional Photo Plethysmography (PPG), not involving speckles, contraction and dilatation of blood vessels as the blood flows through the blood vessels is measured. PPG thus performs a measurement of volume change. However, in conventional Speckle Plethysmography (SPG), wherein speckle contrast is measured, the measurement may be affected also by the movements caused by the blood flow. Therefore, although the term SPG implies measurements of volume change, it should be understood that an SPG signal does not necessarily result solely from volume change but may additionally or alternatively result from other changes of properties such as movements of the tissue and/or changes of scattering properties.

Speckle pattern changes from biological tissue may provide useful information in several medical applications, such as monitoring of vital signs of a person or an animal. Given as non-limiting examples, monitoring speckle pattern changes may provide information on heart rate (HR), heart rate variability (HRV), blood pressure, blood flow, blood volume, cardiac output, arterial elasticity, respiration rate, oxygen saturation, venous and arterial flow pressure motion. However, it should be understood that the use of speckle pattern changes for monitoring is not limited to medical applications. Speckle pattern changes from also other types of biological tissue may provide useful information. By way of example, such biological tissue may be tissue of plants.

The first aspect relates to a method for analyzing a speckle pattern from biological tissue in medical applications. The method may be a computer implemented method for analyzing a speckle pattern from biological tissue.

The method comprises receiving a time sequence of images, wherein each image of the time sequence of images comprises a plurality of elements. By the term "elements" is here meant picture elements that together form the image. This is typically a digital representation of the image, wherein the elements are intended to be arranged in rows and columns to form the image.

It should be understood that when referring to an "image" in the present disclosure, not merely a visually available two-dimensional projection of a three-dimensional object is intended. Rather, it should be seen from a wider point of view in which the digital representation of the elements in e.g. a computer or a processing unit, is also regarded as an image. Further, within the field of digital image processing it is understood by a person skilled in the art that an "image" undergoing processing in e.g. a computer, is not necessarily the visually available representation of the image displayed on screen, but rather it may be the digital representation of the image inside the processing unit that is being processed. In fact, there is no requirement of providing any display of the image on screen for the processing unit to be able to carry out the processing of the image. On the contrary, when a processing unit performs processing of a large amount of images, it is likely to do so without displaying the images on screen. Hence in the present disclosure, included in the term "image" is not only visually available images, but also digital representations of images inside the processing unit or a computer memory.

The image received may comprise a speckle pattern from biological tissue to be analyzed. The speckle pattern may have been acquired in reflection mode or it may have been acquired in transmission mode, as discussed above. Subsequently, filtering and analysis is applied to each respective image of the time sequence of images.

Since the analysis of the method is focused on the speckle pattern itself rather than on the biological tissue imaged, a digital spatial filter procedure is applied to the image for enhancing areas in the image corresponding to speckles of the speckle pattern. The digital spatial filter procedure aims at enhancing the speckle pattern in the image by compensating the image for other variations in light intensity originating from other features than the speckle pattern.

By way of example, intensity variations across the image may be caused by uneven illumination. For example, if a laser beam is used as illumination, the laser beam may feature a Gaussian intensity profile wherein the light intensity is higher in the center of the laser beam than at the outer edges of the laser beam.

By way of further example, intensity variations across the image may be caused by non-homogeneous detection. It is conceivable that for example light collecting optics may result in higher light intensity at a central part of the image as compared to more peripheral parts of the image.

As yet another example, intensity variations across the image may be caused by spatial shapes and structures of the biological tissue being illuminated. A curved surface of biological tissue may result in uneven illumination or may cast shadows with respect to the illuminating light from the light source.

By compensating the image for variations in light intensity originating from such features, by means of the digital spatial filter procedure, an image of the speckle pattern may be provided wherein the speckle pattern may be separated or isolated from such other features. The filtered image may facilitate further analysis of the speckle pattern since intensity variations from other features that may cause interference or errors in further analysis, are reduced or even eliminated.

The digital spatial filter procedure may comprise a single filter, or alternatively a plurality of filters, for enhancing the speckles of the speckle pattern. The present disclosure is not limited to any particular type of filter, but the digital spatial filter procedure may comprise any suitable type of filter for enhancing the speckle pattern. It should be understood that also filters for removing speckle patterns from an image may be used, and the information removed from the image may then be used for further analysis.

Given as a non-limiting example, the digital spatial filter procedure may comprise one or more Gabor filters. A Gabor filter is a spatial bandpass filter operating in the spatial frequency domain. A Gabor filter may be used to detect and/or enhance image features that has a predefined scale, or spatial frequency, and a predefined orientation. By combination of a plurality of Gabor filter, set for a plurality of different orientations, orientation invariant features in the image may be found. Speckles of a speckle pattern are typically considered to be orientation invariant features, as they often take the shape of small dots with no extension in any particular direction.

Given as another non-limiting example, the digital spatial filter procedure may comprise transform domain filter such as discrete wavelet transform. Discrete wavelet transformation may be used as an alternative way of spatial bandpass filtering.

Given as yet another non-limiting example, the digital spatial filter procedure may comprise connected component analysis. Connected component analysis, also referred to as "Blob detection", may be optimized to be selective to bright and dark speckles of the speckle pattern.

It serves to mention that the digital spatial filter procedure may be performed in the spatial domain, or it may alternatively be performed in the spatial frequency domain. In the latter case, by way of example, the digital spatial filter procedure may comprise conversion of the image into the spatial frequency domain, by digital Fourier Transform such as for example two-dimensional digital Fourier transform. One or more frequency filters may be applied to the Fourier Transformed image, in order to enhance the spatial frequencies corresponding to the spatial frequencies of the speckle pattern. Subsequently, the frequency filtered image may be transformed back to the spatial domain by inverse Fourier Transform, to provide the filtered image. However, it is conceivable that the frequency filtered image alternatively forms the filtered image without being transformed back to the spatial domain. In the latter case, the frequency filtered image may subsequently be analyzed to determine a speckle pattern value representing an amount of distinguishable speckles of the speckle pattern.

By "generating a filtered image" is here meant that by applying the digital spatial filter procedure to the image, new values for each element of the filtered image are calculated. In case the digital spatial filtering procedure is applied to the image by a processing unit, the values of the elements forming the filtered image may be stored at least temporarily in the processing unit. Such information enables generation of a visual image, however, the visual image is not necessarily displayed when performing the method.

The filtered image is analyzed to determine a speckle pattern value representing an amount of distinguishable speckles of the speckle pattern.

The speckle pattern value may be determined for at least one region of the filtered image. By way of example, the at least one region may be a single region of the image. The single region may be the full image, or it may be a region smaller than the full image. In case the at least one region is a single region of the filtered image, the speckle pattern value is determined for this single region. By way of further example, the at least one region may be a plurality of regions. In case the at least one region is a plurality of regions, a plurality of speckle pattern values may be determined, thus one speckle pattern value for each respective region of the plurality of regions. Put differently, for each respective region of the plurality of regions, a speckle pattern value may be determined representing an amount of distinguishable speckles of the speckle pattern in that region of the filtered image.

The method is intended for analysis of speckle patterns and as such the received images are expected to comprise a speckle pattern. However, it should be understood that not all images received must necessarily comprise a speckle pattern. Thus, it may occur that some images received may not comprise a speckle pattern. It serves to mention that a problem with conventional speckle contrast techniques such as SPG is that they can produce a false SPG waveform as output even when there are no speckles in the images. This is a problem especially when the SPG is used in transmission mode. One reason for this is that the SPG signal extracted using this method may be corrupted by the PPG signal. However, for the method according to the first aspect, if an image without a speckle pattern is received, the method may detect the absence of speckles. The method is aiming at enhancing the morphology of the speckles of the speckle pattern and isolate the speckles from other intensity variations in the image. However, if no speckle pattern is present in the image, this will not be enhanced, and thus no relevant speckle pattern value will be determined. Thus, no false speckle pattern value will be determined.

An advantage is that since the method isolates the speckle pattern from other light intensity variations across the images, errors in the determination of the speckle pattern values caused by other light intensity variations in the images are reduced or possibly eliminated. In this manner, a method for analysis of speckle patterns from biological tissue with high accuracy may be provided.

Another advantage is that since the method isolates the speckle pattern from other light intensity variations in the images, the method provides a signal only when the image comprises a speckle pattern. In this manner, false signals resulting from e.g. PPG information in the image, may be avoided. By the present arrangement, a more reliable method for speckle analysis than conventional speckle contrast methods may be provided.

According to an embodiment, the analyzing the filtered image to determine the speckle pattern value comprises determining a sum of all values of the at least one region of the filtered image, the sum representing an amount of distinguishable speckles of the speckle pattern.

It should be understood that determining the sum of all values of the elements of the at least one region may be used as the only operation of determining the speckle pattern value, or alternatively determining the sum of all values of the elements of the at least one region may be used in combination with other operations of determining the speckle pattern value. By way of example, determining the sum may be part of determining an average of all values of the at least one region of the filtered image. Determining the average would thus involve determining said sum, followed by division of the total number of values in the at least one region.

As also described above, in case the at least one region is a plurality of regions, each region may be analyzed individually, such that one speckle pattern value may be determined for each respective region of the plurality of regions.

An advantage with this embodiment is that summation of all values in a region is a computationally efficient manner of determining a value representing an amount of distinguishable speckles of the speckle pattern. Thus, the values may be determined at a high speed, and even with low processing power.

It should be construed that the analyzing the filtered image to determine a speckle pattern value may comprise alternative operations to determining the sum of all values. By way of example, analyzing the filtered image to determine a speckle pattern value may further comprise determining an average speckle contrast in the at least one region of the filtered image. Determining an average speckle contrast may comprise determining a plurality of speckle contrasts in a plurality of local neighborhoods in the at least one region of the filtered image, and determining the average speckle contrast by averaging of said plurality of speckle contrasts.

According to an embodiment, the method further comprises:
generating, by means of the speckle pattern value from each respective filtered image of the time sequence of images, a time sequence representing variations of the amount of distinguishable speckles of the speckle pattern in the at least one region.

The time sequence representing variations of the amount of distinguishable speckles of the speckle pattern corresponds to the SPG signal. Thus, the time sequence may provide information on moments of living biological tissue caused by for example blood flow through the tissue and contraction and dilatation of blood vessels in the tissue.

An advantage with this embodiment is that the method isolates the speckle pattern from other light intensity variations in each respective image, when determining the respective speckle contrast values. In this manner, false signals resulting from e.g. PPG information in the image, may be avoided, since the method provides a signal only when the image comprises a speckle pattern. By the present arrangement, a more reliable method for extracting an SPG signal may be provided.

According to an embodiment, the method further comprises:
applying thresholding to the filtered image, wherein each element of the plurality of elements in the filtered image is assigned a thresholded value selected from a group of at least two predefined fixed thresholded values, wherein each element is assigned the thresholded value by comparing a filtered value of the element in the filtered image to at least one threshold value.

Applying thresholding by at least one threshold value, and optionally more threshold values, is a manner of sorting the filtered values of the elements into different groups depending on the values of the elements.

According to an embodiment, the at least one threshold value is a single threshold value, and wherein the thresholding performs a binarization of the filtered image, such that elements with filtered values of the filtered image being above the single threshold value are assigned a thresholded value equal to 1, and elements with filtered values of the filtered image being below the single threshold value are assigned a thresholded value equal to 0.

Applying thresholding by a single threshold value, is a manner of sorting the filtered values of the elements into two different groups corresponding to bright or dark in the image. Bright elements are assigned the value 1 and dark elements are assigned the value 0.

If such binarization were to be carried out without first having applied the digital spatial filter procedure, it is conceivable that e.g. bright spots of the speckle pattern may still be below the threshold value in some parts of the image, due to variations in overall light intensity across the image, and would therefore be assigned the value 0. Further, it is conceivable that e.g. dark spots of the speckle pattern may still be above the threshold value in some other parts of the image, due to variations in overall light intensity across the image, and would therefore be assigned the value 1. Consequently, such thresholding may erroneously result in the elements assigned the value 0 would correspond to some bright speckles and some dark speckles, where only dark speckles was intended. Similarly, such thresholding may erroneously result in the elements assigned the value 1 would correspond to some dark speckles and some bright speckles, where only bright speckles was intended. This approach would not form a suitable basis for distinguishing between bright and dark speckles, and thus not for further speckle analysis.

However, according to the present disclosure, the digital spatial filter procedure is applied so as to mitigate or eliminate intensity variations in the image not originating from the speckle pattern, to balance all values to the same base level. Once the intensity is balanced, a common threshold value may be defined that correctly separates bright and dark spots of the speckle pattern by thresholding.

An advantage with this embodiment is that binarization is an easy and efficient manner of sorting bright and dark elements in an image.

According to an embodiment, the applying a digital spatial filter procedure comprises enhancing orientation invariant features in the image corresponding to speckles of the speckle pattern, thereby generating the filtered image.

Speckles of a speckle pattern are typically considered to be orientation invariant features, as they often take the shape of small bright and dark dots with no extension in any particular direction. This property of the speckles may therefore be a suitable property to isolate when enhancing speckles of a speckle pattern. Other features in the image, such as the biological tissue itself, may typically have much larger size than the speckles and may be extending in a one or more direction.

An advantage with the present embodiment is that enhancing orientation invariant features may be an efficient manner of distinguishing speckles of the speckle pattern from other features in the image.

According to an embodiment, the applying a digital spatial filter procedure comprises applying a plurality of digital spatial filters, sequentially or simultaneously, to the image, thereby generating the filtered image.

Although a single digital spatial filter may provide filtering of the image that enhances the speckles of the speckle pattern, it should be understood that the digital spatial filter procedure may provide better filtering if applying a plurality of digital spatial filter, in that the enhancement of the speckles of the speckle pattern is improved. The plurality of different filters may be the same type of filter, but with different properties in terms of spatial sizes, frequencies, and/or orientation of the individual digital spatial filters. Alternatively, the plurality of digital spatial filters may comprise different types of filters, being able to filter different aspects of image information.

An advantage with this embodiment is that a method with improved filtering may be provided, that may more accurately distinguish between the speckles of the speckle pattern and other intensity variations in the image and thus may more accurately enhance the speckles of the speckle pattern in the image.

According to an embodiment, the plurality of digital spatial filters is a plurality of two-dimensional Gabor filters, wherein each Gabor filter of the plurality of two-dimensional Gabor filters has a unique orientation with respect to the image.

As previously mentioned, speckles of a speckle pattern are typically considered to be orientation invariant features, as they often take the shape of small bright and dark dots with no extension in any particular direction. A Gabor filter is a spatial bandpass filter that may be used to detect and/or enhance image features that has a predefined scale, or spatial frequency, and a predefined orientation. By combination of a plurality of two-dimensional Gabor filter, set for a plurality of different orientations, orientation invariant features in the image, such as speckles, may be detected. By way of example, the digital spatial filter procedure may comprise one, two, three, four, five, six, seven, eight, nine, ten or more Gabor filters. By way of further example, the plurality of Gabor filters may be configured to have orientations that are evenly distributed with respect to angle in the plane of the image. By applying the plurality of Gabor filters with different, unique orientations to the image, and then combining the result of the plurality of Gabor filters into a filtered image, image features that extend in particular directions may be reduced or eliminated, and thus orientation invariant features, such as speckles, may be enhanced.

An advantage with this embodiment is that a method which efficiently enhances the speckles of the speckle pattern in the image may be provided. Gabor filters are easy to implement, and are well suited for filtering images with respect to spatial frequency. The combination of a plurality of Gabor filters may thus provide an efficient manner of enhancing speckles of the speckle pattern.

The plurality of Gabor filter may be configured for a plurality of different orientations, and for a specific spatial size or frequency. However, it serves to mention that the plurality of Gabor filters may be further configure for plurality of different orientation and for a plurality of different spatial sizes or frequencies. In such a configuration, for each spatial size or frequency the digital spatial filter procedure may comprise a number of Gabor filters for different orientations. In this manner, orientation invariant features may be filtered at a plurality of different spatial sized or frequencies.

The present arrangement may be advantageous in situations for example, where the exact size of the speckles is not known beforehand, and/or when the speckles of the speckle pattern may have a range of different sizes.

According to an embodiment, the applying a digital spatial filter procedure comprises individually applying each Gabor filter of the plurality of two-dimensional Gabor filters, sequentially one by one, to the image, and combining the individually Gabor filtered images into the filtered image.

According to an embodiment, the combining comprises multiplying the individually Gabor filtered images with each other to generate the filtered image.

By "multiplying the individually Gabor filtered images with each other" is here meant that the value of an element in one Gabor filtered image is multiplied with the respective values in the respective elements of all other Gabor filtered images corresponding to the same position.

An advantage with this embodiment is that it provides an efficient yet easily implemented manner of combining several individually Gabor filtered image into a filtered image. Picture element multiplication is not computationally demanding and may therefore also be performed at a high rate.

Another advantage is that the operation of multiplication is one manner in which orientation invariant features are enhanced, since only picture elements providing a high response in all orientations are maintained in the filtered image.

According to a second aspect there is provided a computer program product comprising a computer-readable storage medium storing computer-readable instructions which, when executed by a processing unit, will cause the processing unit to perform the method according to the first aspect.

According to a third aspect there is provided a device for analyzing a speckle pattern from biological tissue, the device comprising a processing unit configured to:
receive a time sequence of images of the speckle pattern, wherein each image of the time sequence of images comprises a plurality of elements; and
for each respective image of the time sequence of images:
   apply a digital spatial filter procedure to the image for enhancing areas in the image corresponding to speckles of the speckle pattern, thereby generating a filtered image wherein a morphology of speckles of the speckle pattern is enhanced by compensation for variations in light intensity in the image; and
   analyze the filtered image to determine a speckle pattern value representing an amount of distinguishable speckles of the speckle pattern in at least one region of the filtered image.

The processing unit may be implemented as a general-purpose processing unit, such as a central processing unit (CPU), which may execute the instructions of one or more computer programs in order to implement the method. The processing unit may alternatively be implemented as firmware arranged in an embedded system, or as a specifically designed processing unit, such as an Application-Specific Integrated Circuit (ASIC) or a Field-Programmable Gate Array (FPGA), which may be configured to implement the method.

An advantage with embodiments with integrated solution is that the need for sending and storing the images may be avoided.

Another advantage with integrated solutions is that it may allow on-chip processing of the images to be made, and thus the need for an external computer may be eliminated.

According to an embodiment, the device further comprises a detector configured to detect the time sequence of images of the speckle pattern, wherein the detector is connected to the processing unit and further configured to transmit the time sequence of images to the processing unit.

By the term "detector" is here meant any image detector onto which a plurality of separate light sensitive elements is arranged such that they may individually detect the light intensity impinging onto the respective light sensitive elements, and in response thereof produce an electrical signal. The light sensitive elements on the detector may be arranged along one or more rectilinear lines. By way of example, a detector may be, but is not limited to, a charge-coupled device (CCD) and complementary metal oxide semiconductor (CMOS).

By way of example, each respective light sensitive element may be configured to generate the information for a mutually unique picture element of the image. Alternatively, the light sensitive elements may be configured in clusters, such that each cluster of light sensitive elements may be configured to generate the information for a mutually unique picture element of the image.

The detector is connected to the processing unit such that the detector may transmit the time sequence of images to the processing unit. It is conceivable that there may be a physical connection between the detector and the processing unit. By way of example, a physical connection may be used when the detector and the processing unit are arranged in the same housing and/or part of a common integrated circuit. It is further conceivable that the connection, alternatively, may be a wireless connection allowing the time sequence of images to be wirelessly transmitted to the processing unit. By way of example, a wireless connection may be used when the processing unit is located remotely to the detector.

It serves to mention that the device comprising the processing unit and the detector may not only be used for analysis of speckle pattern from biological tissue, but may also find other applications. By way of example, the device may be used to test laser sources and optical systems to evaluate speckle density, and thereby evaluate operational parameter of the laser source.

According to an embodiment, the device further comprises a light source configured to generate light for illuminating the biological tissue, such that the light is scattered by the biological tissue and forms the speckle pattern.

By the term "light source" is here meant any unit, device and/or element at which light is generated. By way of example, the light source may be, but is not limited to a laser, a laser diode, a light emitting diode, an incandescent light source, a fluorescent light source, or a combination thereof. In this context the term "light" should be allowed a broad interpretation, not limited to visible electromagnetic radiation. Rather, the term "light" may also include for example ultra-violet light and infra-red light.

According to an embodiment, the light source is configured to generate light being at least partially coherent.

Coherent light may be advantageous as it improves the interference and thus the visibility and contrast of the speckles. A coherent light source may be a laser. However, it should be understood that also partially coherent light may provide a speckle pattern with sufficient visibility and contrast. A partially coherent light source may e.g. be a light emitting diode, LED, emitting light through a pinhole onto the biological tissue. A coherent light source may provide better speckle contrast but may be more expensive while a partially coherent light source may provide less speckle contrast but may be less expensive.

According to an embodiment, the light source is configured to generate light of at least two wavelengths.

According to an embodiment, the device further comprises a housing configured to be wearable on a body of a person or an animal, and wherein one or more of the processing unit, the detector, and the light source are arranged in or on the housing.

By way of example, the housing may comprise a textile/garment configured to be worn on the body, or the housing may comprise a belt or strap, configured to be worn around a body part of the body. By way of further example, the housing may be implemented in the form of a clamp configured to be clamped around a body part of the body.

It is conceivable that the detector and the light source are arranged in or on the housing such that illumination of the biological tissue, as well as detection of the time sequence of images of the speckle pattern may be provided in close proximity to the biological tissue. It is further conceivable that the processing unit may be arranged in or on the housing, such that the method for analyzing the speckle pattern from the biological tissue may be performed within the housing. As an alternative, it is conceivable that the processing unit is arranged external to the housing, such that the time sequence of images may be transmitted to a location remote to the housing at which the processing unit is located and such that the method for analyzing the speckle pattern from the biological tissue may be performed externally to the housing.

An advantage with this embodiment is that it may provide a convenient manner of wearing the device, such that the device may be worn for a long time and thus providing monitoring for a long time, while minimizing discomfort for the wearer.

It serves to mention that, as an alternative to the device being configured to be worn on a body of a person or an animal, the device may instead be configured as a device for remote sensing. A device for remote sensing may illuminate the biological tissue and/or acquire images of the speckle pattern at a distance from the body of a person or an animal. When monitoring e.g. vital signs by a device for remote sensing, no contact between the device and the body may be required. By way of example, a device for remote sensing may illuminate and/or acquire images from a distance of about 0.5 to 2 meters.

Effects and features of the second and third aspects are largely analogous to those described above in connection with the first aspect. Embodiments mentioned in relation to the first aspect are largely compatible with the second and third aspects. It is further noted that the disclosure relates to all possible combinations of features unless explicitly stated otherwise.

Other objectives, features and advantages of the present disclosure will appear from the following detailed description, from the attached claims as well as from the drawings.

### Brief descriptions of the drawings

The above, as well as additional objects, features and advantages of the present disclosure, will be better understood through the following illustrative and non-limiting detailed description, with reference to the appended drawings. In the drawings like reference numerals will be used for like elements unless stated otherwise.
Fig. 1 illustrates a schematic block diagram summarizing the method for analyzing a speckle pattern from biological tissue.
Fig. 2A illustrates an example of an original image of a speckle pattern superimposed on an image of biological tissue.
Fig. 2B illustrates an example of a filtered image, after application of the digital spatial filter procedure to the original image.
Fig. 2C illustrates a binarized filtered image.
Fig. 3 schematically illustrates an example of the effect of using a plurality of Gabor filters on an image.
Fig. 4A illustrates results from conventional SPG analysis when images are acquired in reflection mode.
Fig. 4B illustrates results from the method according to the present disclosure when images are acquired in reflection mode.
Fig. 5A illustrates results from conventional SPG analysis when images are acquired in transmission mode, with no speckle pattern present in the image.
Fig. 5B illustrates results from the method according to the present disclosure when images are acquired in transmission mode.
Fig. 6A schematically illustrates a device for analyzing a speckle pattern from biological tissue.
Fig. 6B schematically illustrates a device for remotely analyzing a speckle pattern from biological tissue.

### Detailed description

In cooperation with attached drawings, the technical contents and detailed description of the present inventive concept are described thereinafter according to a preferable embodiment, being not used to limit the claimed scope. This inventive concept may be embodied in many different forms and should not be construed as limited to the embodiments set forth herein; rather, these embodiments are provided for thoroughness and completeness, and fully convey the scope of the inventive concept to the skilled person.

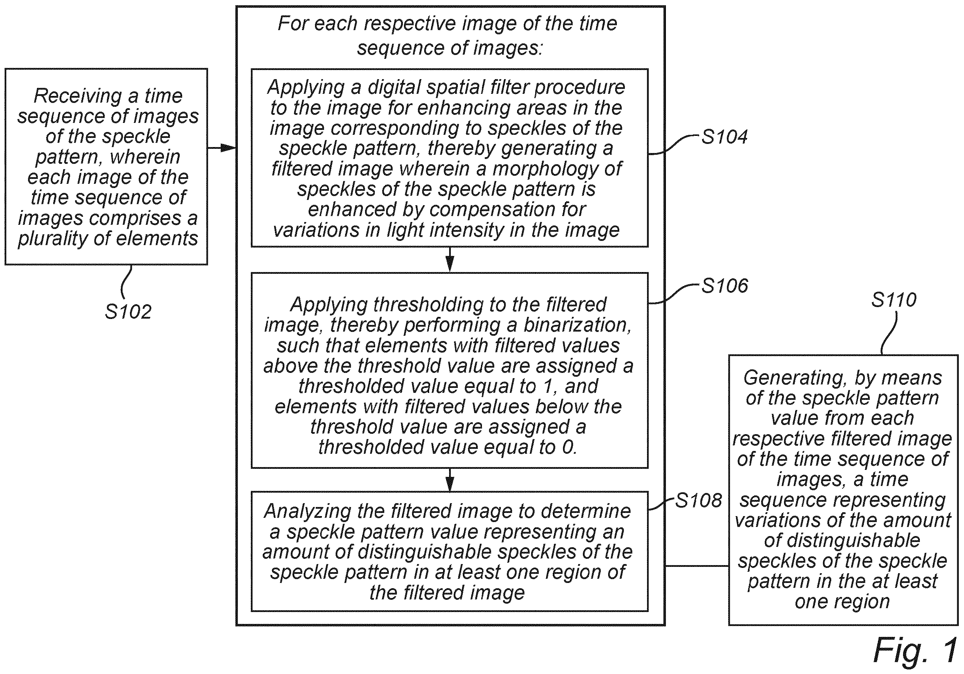

Fig. 1 illustrates a schematic block diagram summarizing the method for analyzing a speckle pattern from biological tissue. It should be understood that the steps of the method, although listed in a specific order herein, may be performed in any order suitable.

The method comprises receiving S102 a time sequence of images of the speckle pattern. The time sequence of images may have been acquired using a detector detecting the time sequence of images of the speckle pattern from biological tissue to be analyzed. The speckles of the speckle pattern are seen as bright and dark dots. The speckle pattern may have been acquired in either reflection mode or in transmission mode. Each image of the time sequence of images comprises a plurality of elements, which may be referred to as picture elements, that together form the image.

By monitoring the speckle pattern, movements of living biological tissue caused by for example blood flow through the tissue and contraction and dilatation of blood vessels in the tissue may be monitored. The speckle pattern is perturbed by the movements, and the speckle pattern is blurred, resulting in fewer distinguishable speckles of the speckle pattern. However, the speckle pattern is superimposed on an image of the biological tissue also featuring light intensity variations. Light intensity variations from other features may cause interference or errors in further analysis of the speckle pattern, and it is therefore desirable to reduce or even eliminate such intensity variations, prior to further analysis.

For each respective image of the time sequence of images, the method comprises applying S104 a digital spatial filter procedure to the image. The digital spatial filter procedure is applied for enhancing areas in the image corresponding to speckles of the speckle pattern, thereby generating a filtered image. In the filtered image a morphology of speckles of the speckle pattern is enhanced by compensation for variations in light intensity in the image, originating from other image features than the speckle pattern. Put differently, the digital spatial filter procedure is applied so as to mitigate or eliminate intensity variations in the image not originating from the speckle pattern, to balance all values to the same base level.

Speckles of a speckle pattern are typically considered to be orientation invariant features, as they are often shaped with no extension in any particular direction. Other features in the image, may have larger size than the speckles and may be extending in a one or more direction. Thus, as an option, the applying S104 a digital spatial filter procedure may comprise enhancing orientation invariant features in the image corresponding to speckles of the speckle pattern, to generate the filtered image.

The applying S104 a digital spatial filter procedure may comprise applying a single digital spatial filter to the image. Alternatively, the applying S104 a digital spatial filter procedure may comprise applying a plurality of digital spatial filters to the image. The plurality of digital spatial filter may be applied either sequentially or simultaneously to the image.

By way of example, the plurality of digital spatial filters may be a plurality of two-dimensional Gabor filters, each of which having a unique orientation with respect to the image. Each Gabor filter may be individually applied, sequentially one by one, to the image. Thus, each Gabor filter may filter out features extending in a specific spatial direction. The individually Gabor filtered images may subsequently be combined to form the filtered image. For example, the individually Gabor filtered images may be combined by multiplying the individually Gabor filtered images with each other to generate the filtered image.

Hence, applying S104 the digital spatial filter procedure to the image may mitigate or eliminate intensity variations in the image not originating from the speckle pattern, such that the intensity variations in the filtered image may originate from the speckle pattern, at least to a major part.

Optionally, the method may further comprise applying S106 thresholding to the filtered image, by comparing the filtered value of each element in the filtered image to a single threshold value. Upon applying S106 thresholding, elements with filtered values above the single threshold value are assigned a thresholded value equal to 1, and elements with filtered values below the single threshold value are assigned a thresholded value equal to 0. In this manner, the thresholding performs a binarization of the filtered image. By the present arrangement, a filtered image may be provided which, after thresholding, may comprise elements with the value of 1 corresponding to a bright speckle of the speckle pattern, and may comprise elements with the value of 0 corresponding to a dark speckle of the speckle pattern.

It serves to mention that binarization of the filtered image is a non-limiting example of how thresholding may be performed. It is conceivable that thresholding may alternatively comprise more than two thresholded values, and/or that these values may be different from the values 1 and 0. Further, thresholding may comprise more than a single threshold value, for example two, three or four threshold values.

The method further comprises analyzing S108 the filtered image to determine a speckle pattern value representing an amount of distinguishable speckles of the speckle pattern in at least one region of the filtered image.

By way of example, the analyzing S108 the filtered image to determine the speckle pattern value may comprise determining a sum of all values of the at least one region of the filtered image. As the filtered image has been filtered and binarized, all elements with value of 1 may represent the bright speckles in the region. Thus by summarizing all elements, the speckles are counted. The summarizing the values of the elements may optionally be followed by division of the total number of values in the region, thereby determining the average of the values. In either of these cases, the speckle pattern value may represent an amount of distinguishable speckles of the speckle pattern.

Optionally, the method may further comprise generating S110 a time sequence of the speckle pattern values from each respective filtered image of the time sequence of images. The time sequence of speckle pattern values may represent variations of the amount of distinguishable speckles of the speckle pattern in the region.

The time sequence corresponds to an SPG signal. Thus, the time sequence may provide information on movements of living biological tissue caused by for example blood flow through the tissue and contraction and dilatation of blood vessels in the tissue.

However, as opposed to conventional SPG, the method provides a signal only when the image comprises a speckle pattern. Since the method isolates the speckle pattern from other light intensity variations in each respective image, when determining the respective speckle contrast values, false signals resulting from e.g. PPG information in the image, may be avoided. Thus, a more reliable method for extracting an SPG signal is provided.

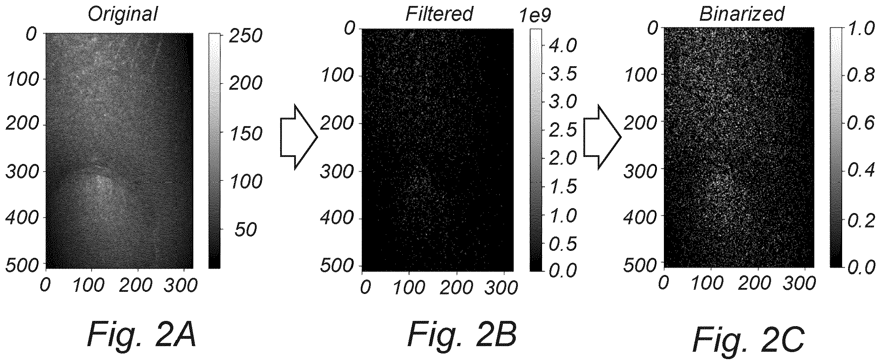

Fig. 2 illustrates an example of how the speckle pattern is enhanced by the method. An original image, Fig. 2A, is received as one of the images in the time sequence of images. The original image comprises a speckle pattern superimposed on an image of biological tissue, in the present case a finger. The speckle pattern is seen as bright and dark dots. However, also other intensity variation are seen across the image. At least some of these intensity variations are due to the shape of the finger and the nail. Some areas are overall brighter than others. It can be realized that some bright speckle dots in the dark areas are not as light intense as some dark speckle dots in the bright areas. Hence, it may be challenging to distinguish between bright and dark speckles simply by analyzing the intensity of the picture elements in the image.

Fig. 2B illustrates an example of a filtered image, after application of the digital spatial filter procedure to the original image of Fig. 2A. Light intensity differences across the image due to for example the shape of the finger and the nail have been compensated for, such that what remains in the filtered images are substantially the intensity variations of the speckle pattern. The bright speckles are seen as bright dots across a dark background the finger is no longer visible. The speckles are thus clearly distinguishable from other features of the original image.

In Fig. 2B the elements with bright dots may have different values, thus different intensities. As mentioned in relation to Fig. 1, the method may optionally comprise thresholding or binarization of the filtered image. Fig. 2C illustrates such a binarized filtered image, in which all the bright dots have been assigned the value of 1 and the remaining dark dots or areas have been assigned the value of 0.

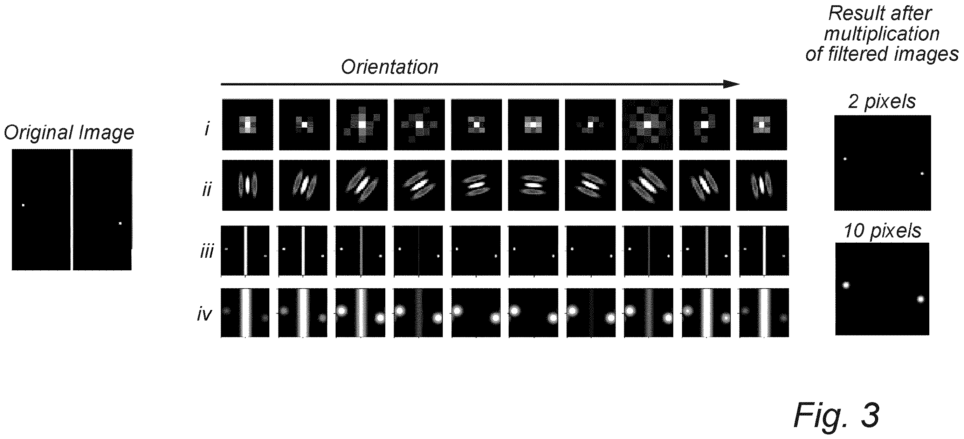

Fig. 3 schematically illustrates an example of the effect of using a plurality of Gabor filters on an image. On the left hand side, an original image is shown, serving as input to the digital spatial filter procedure. The image comprises two white dots and one vertical white line on a black background. The two dots may be considered to correspond to speckles, and the vertical line may be considered to correspond to a larger feature in the image.

The uppermost line i) illustrates a plurality of two-dimensional Gabor filters, in the present example ten Gabor filters. Each Gabor filter has a unique orientation with respect to the image. The first Gabor filter in line i) has a horizontal orientation, such that it may identify spatial frequencies in the horizontal direction. The orientation is shifted with an angle with respect to the horizontal orientation for each consecutive Gabor filter, for identification of spatial frequencies in other directions of the image. The spatial size or frequency of the filters corresponds to 2 picture elements.

Line ii) illustrates another plurality of Gabor filters, similar to that of line i). However, the spatial size or frequency of the filters in line ii) corresponds to 10 picture elements.

Line iii) illustrates the individually Gabor filtered images resulting from applying the respective filters of line i) onto the original image. As can be seen in line iii), the two dots as well as the vertical line are clearly visible in the first image resulting from the use of the Gabor filter with horizontal orientation. Thus, the horizontal Gabor filter identifies the spatial frequencies in the horizontal direction for these image features. However, for the consecutive filters, for which the angle deviates from the horizontal direction, the vertical line gradually fades away, and when applying filters with substantially vertical orientation, i.e. perpendicular to the horizontal direction, the vertical line is not visible. In other words, since the line extends in the vertical direction there is no spatial frequency of the defined size in the vertical direction. The two dots, on the other hand, are visible in all individually Gabor filtered images. The dots are thus orientation invariant features, and have the same spatial frequency in all directions in the image. The filtered image, illustrated on the right hand side, is obtained by multiplying, picture element by picture element, the ten individually Gabor filtered images. Since the line is not present in all individually Gabor filtered images, it will not be included in the filtered image.

Line iv) illustrates the individually Gabor filtered images resulting from applying the respective filters of line ii) onto the original image, thus having a spatial size or frequency of the filters corresponding to 10 picture elements. Despite this difference, a similar result is seen as compared to the result using the filters with smaller size. The filtered image also for the second plurality of filters, illustrated on the right hand side, is obtained by multiplying, picture element by picture element, the ten individually Gabor filtered images. Again, since the line is not present in all individually Gabor filtered images, it will not be included in the filtered image.

Hence, in the manner described above, the white dots corresponding to speckles may be efficiently enhanced with respect to other larger features in the image, such as the vertical line.

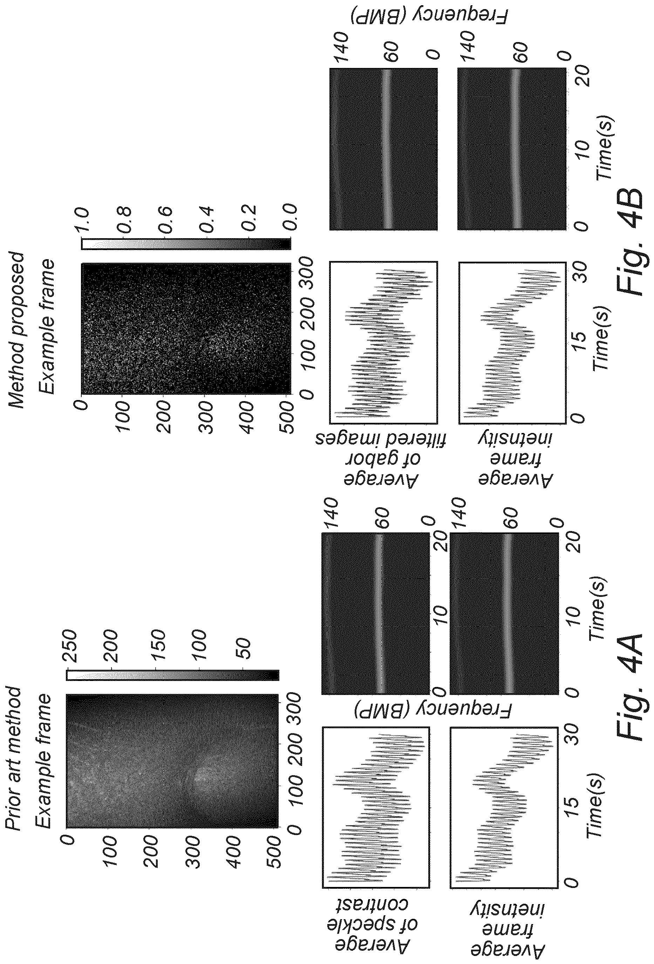

Figs 4A-4B illustrate a comparison between the result from conventional SPG analysis and the result from the method of the present disclosure. In the example of Figs 4A-4B, a detector was positioned to visualize a fingertip of a patient, illuminated by a light source in the form of a laser in reflection mode. Thus, the detector and the light source are arranged on the same side of the finger. In the present example, speckles are formed and imaged by the detector.

Fig. 4A illustrates the results from conventional SPG analysis. At the top, an example image from the time sequence of images is shown.

In conventional SPG analysis, an average speckle contrast is determined in the at least one region of the filtered image, and in the present example in the full image. Determining an average speckle contrast typically comprise determining a plurality of speckle contrasts in a plurality of local neighborhoods in the image. By way of example, a local neighborhood may have the size of 2x2, 3x3, 4x4, or 5x5 picture elements. Typically, the speckle contrast of a local neighborhood is determined by calculating the standard deviation in the local neighborhood of a picture element, and subsequently divide the standard deviation by the average intensity of that neighborhood. The average speckle contrast is determined by averaging of said plurality of speckle contrasts of the local neighborhoods. In the present manner, a single value for the image is calculated.

The calculation of the average speckle contrast is performed for each of the images in the time sequence of images and from these average speckle contrasts, an SPG time domain signal and time-frequency analysis may be plotted, as illustrated in the middle row of Fig. 4A. At the bottom of Fig. 4A, the corresponding PPG time domain signal and time-frequency analysis is illustrated for reference.

Fig. 4B illustrates the results from the method according to the present disclosure. The method is applied to the same time sequence of images as for Fig. 4A, form comparison. At the top, an example filtered image from the time sequence of images is shown. A digital spatial filter procedure has been applied to the original image, according to the method of the present disclosure, thereby providing a filtered image. Thus, the speckles of the speckle pattern are enhanced with respect to other features of the image.

Analysis of the filtered image to determine a speckle pattern value is performed to each respective filtered image of the time sequence. From these speckle pattern values, a time sequence may be generated representing variations of the amount of distinguishable speckles of the speckle pattern, which corresponds to an SPG time domain signal, as illustrated in the middle row of Fig. 4B. Further, also a time-frequency analysis may be plotted, as illustrated. At the bottom of Fig. 4B, the corresponding PPG time domain signal and time-frequency analysis is illustrated for reference.

In the present example, both the conventional SPG analysis and the method of the present disclosure correctly generate an SPG waveform with the right frequency content.

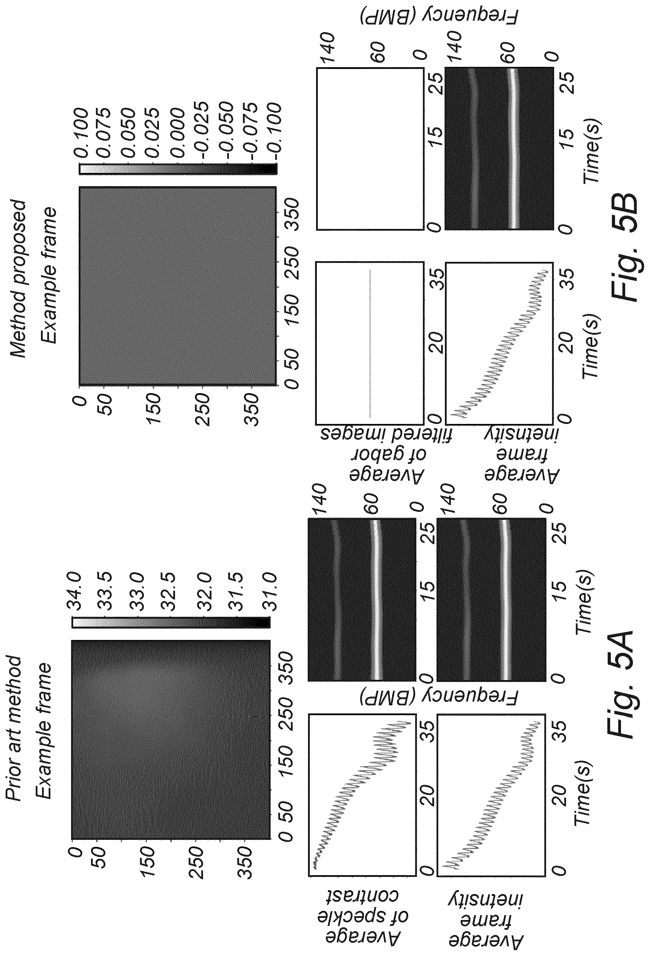

Figs 5A-5B illustrate a comparison between the result from conventional SPG analysis and the result from the method of the present disclosure. In the example of Figs 5A-5B, a detector was positioned to visualize a fingertip of a patient, illuminated by a light source in the form of a laser in transmission mode. Thus, the detector and the light source are arranged on opposite sides of the finger.

It serves to mention that the illumination power required to form a speckle pattern in transmission mode is typically higher than required for the corresponding situation in reflection mode. The reason for this requirement is at least partially due to multiple scattering of the light as it travels through the biological tissue, with the effect that smearing out the coherence of the light and thus the otherwise distinct pattern of bright and dark dots. A speckle pattern may be formed if a high enough laser power is used, but it is nevertheless challenging. In the present example, however, the laser power is not sufficient, and no speckles are observable in the images.

Fig. 5A illustrates the results from conventional SPG analysis. At the top, an example image from the time sequence of images is shown. Since the laser power is not sufficient, the image does not comprise any speckle pattern.

The calculation of the average speckle contrast is performed for each of the images in the time sequence of images and from these average speckle contrasts, an SPG time domain signal and time-frequency analysis may be plotted, as illustrated in the middle row of Fig. 5A. At the bottom of Fig. 5A, the corresponding PPG time domain signal and time-frequency analysis is illustrated for reference.

In the present example, even though the images do not comprise any speckle pattern, an SPG waveform is generated, as illustrated in the middle row of Fig. 5A. However, since there is no speckle pattern in the image, the SPG signal is a false SPG signal, that may be a result from e.g. the PPG information in the image.

Fig. 5B illustrates the results from the method according to the present disclosure. The method is applied to the same time sequence of images as for Fig. 5A, form comparison. At the top, an example filtered image from the time sequence of images is shown. No enhancement of speckles is seen in the filtered image since no speckles are present in the original image.

Analysis of the filtered image to determine a speckle pattern value is performed to each respective filtered image of the time sequence, and a time sequence is generated representing variations of the amount of distinguishable speckles of the speckle pattern, which corresponds to an SPG time domain signal, as illustrated in the middle row of Fig. 5B. No variations over time in the amount of distinguishable speckles is detected, since no speckle pattern is present in the images. No false SPG signal is generated. On the contrary, absence of speckle patterns may be detected. Hence, the method of the present disclosure may solve the problem of false SPG signals by correctly detecting an empty or noisy SPG without any relevant frequency content if no speckles are present.

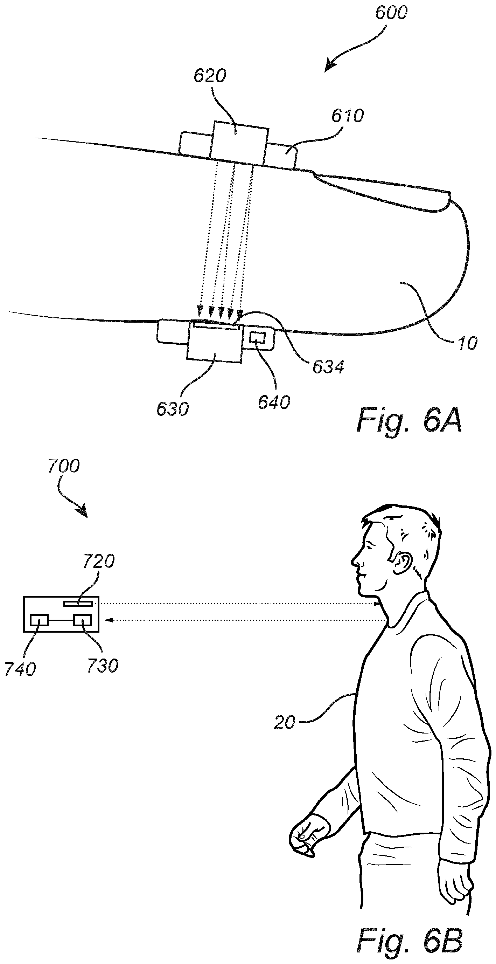

Fig. 6A schematically illustrates a device 600 for analyzing a speckle pattern from biological tissue. In the present example, the biological tissue is a finger 10. The device 600 comprises a housing 610 configured to be wearable on the finger 10. The housing 610 is implemented in the form of a clamp or a finger cuff to be clamped around the finger 10.

The device 600 further comprises a light source 620. The light source 620 is configured to generate light for illuminating through the finger 10. The light source 620 is arranged in the housing 610 such that the light generated by the light source 620 is directed towards a side of the finger 10, when the device 600 is attached to the finger 10. By the present arrangement, the light source 620 may illuminate through the finger 10, such that the light forms the speckle pattern on an opposite side of the finger 10.

The device 600 further comprises a detector 630. The detector 630 is an imaging detector comprising a plurality of separate light sensitive elements 634 arranged such that they may individually detect the light intensity impinging onto the respective light sensitive elements 634. As such, the detector 630 is configured to acquire time sequences of images. The detector 630 is arranged in the housing 610 on an opposite side with respect to the light source 620. The detector 630 is arranged in the housing 610 such that when the device 600 is attached to the finger 10, the light sensitive elements 634 are facing an area of the finger 10 at which light from the light source 610 exits the finger 10. By the present arrangement, the detector 630 may acquire time sequences of speckle patterns formed at a side of the finger 10 opposite to the light source 620. Each image of the time sequence of images comprises a plurality of elements, i.e. picture elements, that together form the image.

The device 600 further comprises a processing unit 640. The processing unit 640 is illustrated in Fig. 6A to be integrated into the housing 610 of the device 600. However, it is conceivable that the processing unit 640 may alternatively be arranged externally to the housing 610, such as in an external computer.

The detector 630 is connected to the processing unit 640 and further configured to transmit the time sequence of images to the processing unit 640.

The processing unit 640 is configured to perform the method as previously disclosed. Thus, the processing unit 640 is configured to receive the time sequence of images of the speckle pattern from the detector 630.

For each respective image of the time sequence of images received, the processing unit 640 is configured to apply a digital spatial filter procedure to the image for enhancing areas in the image corresponding to speckles of the speckle pattern. By application of the digital spatial filter procedure, the image information is compensated for variations in light intensity in the image not originating from the speckle pattern. In this manner, a filtered image is generated wherein the morphology of speckles of the speckle pattern is enhanced.

For each respective image, the processing unit 640 is further configured to analyze the filtered image to determine a speckle pattern value representing an amount of distinguishable speckles of the speckle pattern in at least one region of the filtered image.

By the present arrangement, speckle pattern changes from the finger 10 may be monitored, which may provide useful information in several medical applications, such as vital signs of the patient.

Fig. 6B schematically illustrates a device 700 for remotely analyzing a speckle pattern from biological tissue. In the present example, the biological tissue may be any visible part of the body 20. The device 700 is configured for remote sensing of vital signs of the body 20. By way of example, the device 700 may be configured to be arranged at a distance of 0.5 m to 2 m from the body 20.

The device 700 comprises a light source 720. The light source 720 is configured to generate light for illuminating the body 20. The device 700 may be arranged such that the light generated by the light source 720 is directed towards the body 20. Light illuminating the body 20 may be scattered by the body 20 forming a speckle pattern on the body 20. At least some of the light forming the speckle pattern may be scattered back towards the device 700.

The device 700 further comprises a detector 730. The detector 730 is configured to acquire time sequences of images of the speckle pattern formed on the body 20 and scattered back to the device 700.

The device 700 further comprises a processing unit 740. The processing unit 740 is illustrated in Fig. 6B to be integrated into the device 700. However, it is conceivable that the processing unit 740 may alternatively be arranged externally to the device 700, such as in an external computer.

The detector 730 is connected, either physically or wirelessly, to the processing unit 740, to transmit the time sequence of images to the processing unit 740. The processing unit 740 is configured to perform the method as previously disclosed.

By the present arrangement, speckle pattern changes from the body 20 may be monitored remotely, which may provide useful information of e.g. vital signs of the patient, without the need for physical contact with the patient.

It serves to mention that the processing units 640, 740 do not necessarily need to be permanently configured to perform the method. By way of example, the processing units 640, 740 may be caused to perform the method by a computer program product comprising a computer-readable storage medium storing computer-readable instructions executed by the processing units 640, 740.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A method for analyzing a speckle pattern from biological tissue, the method comprising:
receiving a time sequence of images of the speckle pattern, wherein each image of the time sequence of images comprises a plurality of elements; and
for each respective image of the time sequence of images:
applying a digital spatial filter procedure to the image for enhancing areas in the image corresponding to speckles of the speckle pattern, thereby generating a filtered image wherein a morphology of speckles of the speckle pattern is enhanced by compensation for variations in light intensity in the image; and
analyzing the filtered image to determine a speckle pattern value representing an amount of distinguishable speckles of the speckle pattern in at least one region of the filtered image.

2. The method according to claim 1, wherein the analyzing the filtered image to determine the speckle pattern value comprises determining a sum of all values of the at least one region of the filtered image, the sum representing an amount of distinguishable speckles of the speckle pattern.

3. The method according to any one of claims 1 or 2, further comprising:
generating, by means of the speckle pattern value from each respective filtered image of the time sequence of images, a time sequence representing variations of the amount of distinguishable speckles of the speckle pattern in the at least one region.

4. The method according to any one of the preceding claims, further comprising:
applying thresholding to the filtered image, wherein each element of the plurality of elements in the filtered image is assigned a thresholded value selected from a group of at least two predefined fixed thresholded values, wherein each element is assigned the thresholded value by comparing a filtered value of the element in the filtered image to at least one threshold value.

5. The method according to claim 4, wherein the at least one threshold value is a single threshold value, and wherein the thresholding performs a binarization of the filtered image, such that elements with filtered values of the filtered image being above the single threshold value are assigned a thresholded value equal to 1, and elements with filtered values of the filtered image being below the single threshold value are assigned a thresholded value equal to 0.

6. The method according to any one of the preceding claims, wherein the applying a digital spatial filter procedure comprises enhancing orientation invariant features in the image corresponding to speckles of the speckle pattern, thereby generating the filtered image.

7. The method according to any one of the preceding claims, wherein the applying a digital spatial filter procedure comprises applying a plurality of digital spatial filters, sequentially or simultaneously, to the image, thereby generating the filtered image.

8. The method according to claim 7, wherein the plurality of digital spatial filters is a plurality of two-dimensional Gabor filters, wherein each Gabor filter of the plurality of two-dimensional Gabor filters has a unique orientation with respect to the image.

9. The method according to claim 8, wherein the applying a digital spatial filter procedure comprises individually applying each Gabor filter of the plurality of two-dimensional Gabor filters, sequentially one by one, to the image, and combining the individually Gabor filtered images into the filtered image.

10. The method according to claim 9, wherein the combining comprises multiplying the individually Gabor filtered images with each other to generate the filtered image.

11. A computer program product comprising a computer-readable storage medium storing computer-readable instructions which, when executed by a processing unit, will cause the processing unit to perform the method according to any one of the preceding claims.

12. A device for analyzing a speckle pattern from biological tissue, the device comprising a processing unit configured to:
receive a time sequence of images of the speckle pattern, wherein each image of the time sequence of images comprises a plurality of elements; and
for each respective image of the time sequence of images:
apply a digital spatial filter procedure to the image for enhancing areas in the image corresponding to speckles of the speckle pattern, thereby generating a filtered image wherein a morphology of speckles of the speckle pattern is enhanced by compensation for variations in light intensity in the image; and
analyze the filtered image to determine a speckle pattern value representing an amount of distinguishable speckles of the speckle pattern in at least one region of the filtered image.

13. The device according to claim 12, further comprising a detector configured to detect the time sequence of images of the speckle pattern, wherein the detector is connected to the processing unit and further configured to transmit the time sequence of images to the processing unit.

14. The device according to claim 13, further comprising a light source configured to generate light for illuminating the biological tissue, such that the light is scattered by the biological tissue and forms the speckle pattern.

15. The device according to claim 14, further comprising a housing configured to be wearable on a body of a person or an animal, and wherein one or more of the processing unit, the detector, and the light source are arranged in or on the housing.
